# EUROPEAN PATENT SPECIFICATION

(11) **EP 0 579 733 B1**
(45) Date of publication and mention of the grant of the patent: **20.06.2001**
(21) Application number: 92910028.7
(22) Date of filing: 06.04.1992
(51) Int. Cl.: C07D 417/12, A61K 31/44

(54) **THIAZOLIDINEDIONE DERIVATIVES, PRODUCTION AND USE THEREOF**
THIAZOLIDINDIONDERIVATE, HERSTELLUNG UND ANWENDUNG
DERIVES DE THIAZOLIDINEDIONE, PRODUCTION ET UTILISATION DESDITS DERIVES

(30) Priority: 11.04.1991 JP 7883691
(43) Date of publication of application: 26.01.1994
(73) Proprietor: PHARMACIA & UPJOHN COMPANY, Kalamazoo, Michigan 49001 (US); TAKEDA CHEMICAL INDUSTRIES, LTD., Chuo-ku, Osaka 541 (JP)
(72) Inventor: SOHDA, Takashi, TakaTsuki, Osaka 569 (JP); IKEDA, Hitoshi, Higashiosaka, Osaka 578 (JP); GREENFIELD, John, C., Richland, MI 49083 (US); COLCA, Jerry, R., Texas Township, MI 49009 (US); PETZOLD, Edgar, N., Plainwell, MI 49080 (US)
(74) Representative: Perry, Robert Edward
(86) International application number: US9202566
(87) International publication number: WO9218501

(56) References cited:
- EP-A- 0 008 203
- EP-A- 0 193 256
- Chemical and Pharmaceutical Bulletin, vol. 30, no. 10, October 1982, (Tokyo, JP), T. SOHDA et al.: "Studies on antidiabetic agents. II. Synthesis of 5-[4-(1-methylcyclohexylmethoxy)-benzyl]thiazolidine-2,4-dione (ADD-3878) and its derivatives", pages 3580-3600, see pages 3585-3589
- Arzneim.-Forsch./Drug Res. 40 (I), 1, (1990)

## Description

### FIELD OF THE INVENTION

The present invention relates to novel thiazolidinedione derivatives having hypoglycemic and hypolipidemic activities, to their production and to their use for the manufacture of a medicament for use in treating diabetes.

### BACKGROUND OF THE INVENTION

Various biguanide compounds and sulfonylurea compounds have been used as agents for treating diabetes. However, at present, biguanide compounds are scarcely used because they cause lactic acidosis. Although sulfonylurea compounds have hypoglycemic activity, they often cause serious hypoglycemia and they must be used with caution.

EP-A-0193256 and Chem. Pharm. Bull. 30(10):3580-3600 (1982) disclose thiazolidinediones and their use for the treatment of diabetes. Sohda et al, Drug Res. 40(I)1:37-42 (1990), discloses thiazolidinediones, including 5-{4-[2-(6-hydroxymethyl-2-pyridyl)ethoxy]benzyl}-2,4-thiazolidinedione, and their activity as hypoglycemic agents.

### SUMMARY OF THE INVENTION

According to the present invention, a novel thiazolidinedione derivative is of the general formula (I) 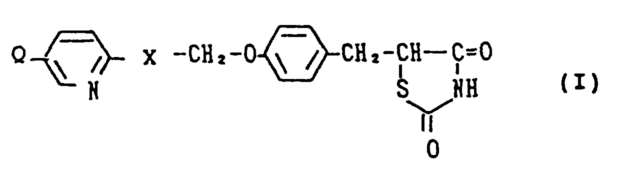 wherein X is -CO- and Q is CH₃CH₂-, or X is -CH₂- and Q is CH₃CO-, CH₃CH(OR)- or -CH₂COOH, wherein R is H or an acyl group selected from formyl, C₂₋₆ alkylcarbonyl, C₈₋₉ aralkylcarbonyl and C₇₋₈ arylcarbonyl, wherein the aralkyl or aryl group optionally has one or more substituents selected from halogen, C₁₋₄ alkoxy and CF₃; or a pharmaceutically-acceptable salt thereof. The compound may be provided in the form of a pure stereoisomer.

The present invention also provides a pharmaceutical composition for treating diabetes, comprising a compound of formula (I) or salt thereof.

One preference is that X is -CH₂- and Q is CH₃CO- or CH₃CH(Oacyl)-, or X is -CO- and Q is CH₃CH₂-. Another preference is that X is -CH₂- and Q is CH₃CH(OH)- or -CH₂COOH.

### DETAILED DESCRIPTION OF THE INVENTION

Examples of the acyl group represented by R in the general formula (I) include formyl, acetyl, propionyl, isobutyryl, pentanoyl, isopentanoyl, hexanoyl, phenylacetyl, phenylpropionyl, benzoyl and p-toluoyl. The aralkylcarbonyl and arylcarbonyl may have one or more substituents such as fluorine, chlorine, bromine, methoxy, ethoxy or trifluoromethyl.

The thiazolidinedione derivative of the general formula (I) (hereinafter referred to as the compound (I)) possesses an acidic nitrogen atom in the thiazolidine ring and a basic nitrogen atom in the pyridine ring. Therefore, the compound (I) of the present invention can exist as both its acidic and basic salts. Examples of the acidic salt include salts with inorganic acids such as hydrochloric acid, hydrobromic acid, sulfuric acid, phosphoric acid and the like, and salts with organic acids such as methanesulfonic acid, toluenesulfonic acid, oxalic acid, malonic acid, maleic acid, fumaric acid, succinic acid, tartaric acid, malic acid and the like. Examples of the basic salt include pharmacologically acceptable salts such as sodium salt, potassium salt, aluminium salt, magnesium salt, calcium salt and the like.

Specific examples of the compound (I) are as follows:
5-[4-[2-(5-acetyl-2-pyridyl)ethoxy]benzyl]-2,4-thiazolidinedione;
5-[4-[2-[5-(1-hydroxyethyl)-2-pyridyl]ethoxy]-benzyl]-2,4-thiazolidinedione;
5-[4-[2-[5-(1-acetoxyethyl)-2-pyridyl]ethoxy]-benzyl]-2,4-thiazolidinedione;
5-[4-[2-[5-(1-propionyloxyethyl)-2-pyridyl]ethoxy]-benzyl]-2,4-thiazolidinedione;
5-[4-[2-[5-(1-butyryloxyethyl)-2-pyridyl]ethoxy]-benzyl]-2,4-thiazolidinedione;
5-[4-[2-[5-(1-isobutyryloxyethyl)-2-pyridyl]-ethoxy]benzyl]-2,4-thiazolidinedione;
5-[4-[2-[5-(1-valeryloxyethyl)-2-pyridyl]ethoxy]-benzyl]-2,4-thiazolidinedione;
5-[4-[2-[5-(1-isovaleryloxyethyl)-2-pyridyl]-ethoxy]benzyl]-2,4-thiazolidinedione;
5-[4-[2-[5-(1-pivaloyloxyethyl)-2-pyridyl]-ethoxy]benzyl]-2,4-thiazolidinedione;
5-[4-[2-[5-(1-benzoyloxyethyl)-2-pyridyl]-ethoxy]benzyl]-2,4-thiazolidinedione;
5-[4-[2-(5-carboxymethyl-2-pyridyl)ethoxy]benzyl]-2,4-thiazolidinedione; and
5-[4-[2-(5-ethyl-2-pyridyl)-2-oxoethoxy]benzyl]-2,4-thiazolidinedione.

The compound (I), the pharmacologically acceptable salt thereof, or the pure stereoisomeric form thereof has hypoglycemic and hypolipidemic activities. Further, the compound (I), the pharmacologically acceptable salt thereof or the pure stereoisomeric form thereof has low toxicity. For example, even when the compound obtained in Example 1 or 2 hereinafter was orally administered to mice in a dose of 300 mg/kg, no lethal case was observed. Therefore, the compound (I), its pharmacologically acceptable salt or pure stereoisomeric form can be used for treatment of diabetes of mammals including man as it is or by combining with a known pharmacologically acceptable carrier, excipient, filler and the like.

Normally, the compound (I), its pharmacologically acceptable salt, stereoisomeric form can be administered orally in the form of, for example, tablets, capsules including soft capsules and micro capsules, powders, granules or the like. If necessary, it can also be administered parenterally in the form of injectable solutions, suppositories, pellets or the like. In the case of oral administration, preferably, it is administered one to three times a day in a daily dose of 0.05 to 10 mg/kg.

The compound (I) and its salt of the present invention can be produced as follows.
(1) Production of the compound (I) wherein X is -CH₂- and Q is CH₃CH(OH)-
   This compound (I-1) can be produced according to the Scheme 1. 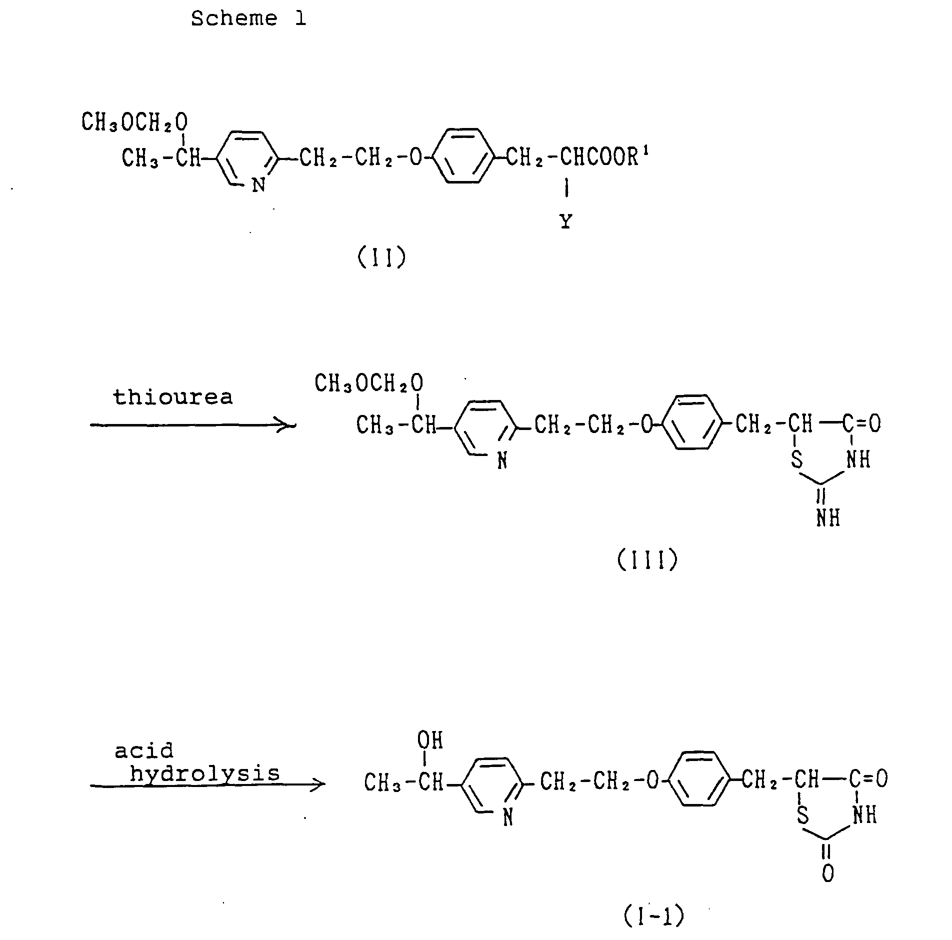 wherein R¹ is a hydrogen atom or a lower alkyl group, and Y is a leaving group.
   Examples of the lower alkyl group represented by R¹ include those having 1 to 4 carbon atoms such as methyl, ethyl, propyl, isopropyl, butyl and the like. In particular, lower alkyl groups having 1 to 3 carbon atoms are preferred. Examples of the leaving group represented by Y include halogen atoms (e.g., chlorine, bromine, iodine), and the like.
   The reaction of the compound (II) with thiourea is normally carried out in a solvent such as alcohols (e.g., methanol, ethanol, propanol, 2-propanol, butanol, isobutanol, 2-methoxyethanol or the like), dimethyl sulfoxide, sulfolane or the like. The reaction temperature is normally 20 to 180°C, preferably 50 to 150°C. The amount of thiourea to be used is 1 to 2 mol per 1 mol of the compound (II). In this reaction, hydrogen bromide is generated as a by-product as the reaction proceeds. Then, the reaction can be carried out in the presence of a deacidification agent such as sodium acetate, potassium acetate or the like to trap the hydrogen bromide. The deacidification agent is normally used 1 to 1.5 mol per 1 mol of the compound (II). The compound (III) is produced by this reaction. The compound (III) can optionally be isolated, but it can be used without isolation in the next acid hydrolysis step.
   Hydrolysis of the compound (III) is normally carried out in a suitable solvent in the presence of water and a mineral acid. Examples of the solvent include the solvents used in the reaction of the compound (II) with thiourea. Examples of the mineral acid include hydrochloric acid, hydrobromic acid, sulfuric acid and the like. The amount of the mineral acid to be used is 0.1 to 20 mol, preferably 0.2 to 10 mol per 1 mol of the compound (III). The water is normally added in large excess. This reaction is normally carried out under warming or heating. The reaction temperature is normally 60 to 150°C. The heating time is normally several hours to twenty and several hours.
(2) Production of the compound (I) wherein X is -CH₂- and Q is CH₃CO-
   This compound (I-2) can be produced by according to the Scheme 2. 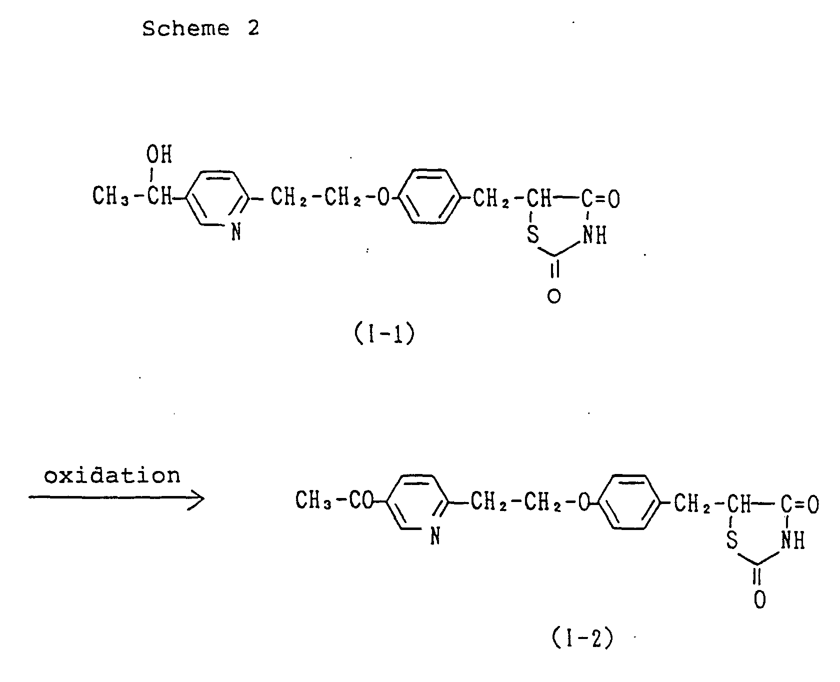
   The oxidation can be carried out according to a known method. Examples thereof include oxidation with manganese dioxide, oxidation with chromic acid (e.g., chromium (IV) oxide-pyridine complex), oxidation with dimethyl sulfoxide and the like [see Shin Jikken Kagaku Koza, Vol. 15 (I-1), (I-2), edited by Japan Chemical Society, Maruzen Shuppan, 1976].
   For example, in the case of the oxidation with dimethyl sulfoxide, the oxidation is normally carried out in an inert solvent such as chloroform, dichloromethane, benzene, toluene or the like. Dimethyl sulfoxide may be used as the solvent. The oxidation is carried out in the presence of an electrophilic reagent such as acetic anhydride, phosphoric anhydride, dicyclocarbodiimide, chlorine or the like. The reaction is normally carried out at about -100 to +60°C. The reaction time is about 0.5 to 50 hours.
(3) Production of the compound (I) wherein X is -CH₂- and Q is CH₃CH(OR)- and R is other than a hydrogen atom
   This compound (I-3) can be produced according to the Scheme 3. 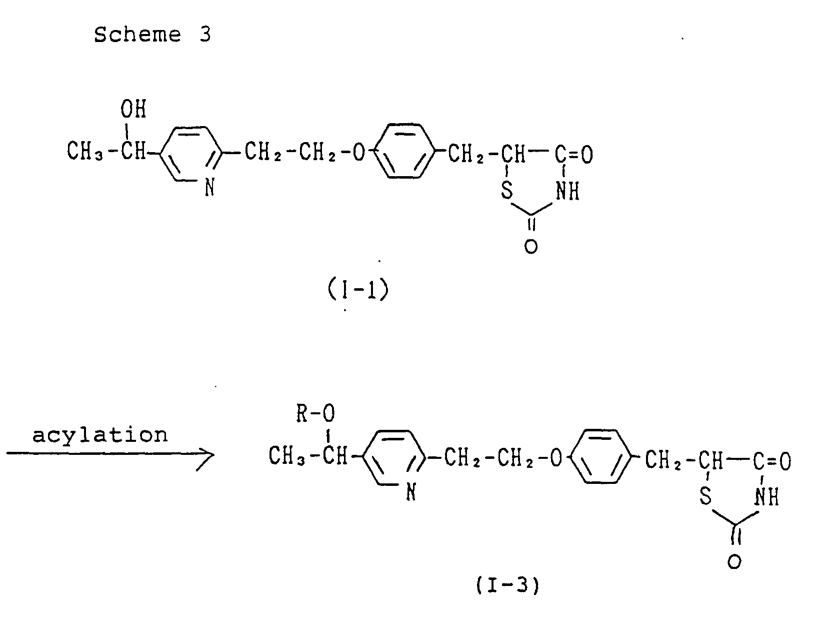
   This acylation is normally carried out by reacting the compound (I-1) with an acylating agent in a suitable solvent in the presence of a base. Examples of the solvent include esters such as ethyl acetate and the like, aromatic hydrocarbons such as benzene, toluene, xylene and the like, ethers such as diethyl ether, diisopropyl ether, tetrahydrofuran, dioxane and the like, ketones such as acetone, methyl ethyl ketone and the like, chlorinated hydrocarbons such as dichloromethane, chloroform, carbon tetrachloride and the like, dimethylformamide and the like. Examples of the acylating agent include formic acid and acid anhydrides and acid halides derived from aliphatic, aryl aliphatic and aromatic carboxylic acids. Examples of the aliphatic carboxylic acid include those having 2 to 6 carbon atoms such as acetic acid, propionic acid, butyric acid, isobutyric acid, valeric acid, isovaleric acid, hexanoic acid and the like. Examples of the aryl aliphatic carboxylic acid include those having 8 to 9 carbon atoms such as phenylacetic acid, phenylpropionic acid and the like. Examples of the aromatic carboxylic acid include those having 7 to 8 carbon atoms such as benzoic acid and the like. These aromatic ring can be substituted by one or more substituents such as halogen atom (fluorine, chlorine, bromine or the like), lower alkoxy having 1 to 4 carbon atoms (e.g., methoxy, ethoxy or the like), trifluoromethyl or the like.
   The amount of the acylating agent to be used is normally 1 to 10 mol, preferably 1 to 2 mol per 1 mol of the compound (I-1). Examples of the base include amines such as pyridine, triethylamine and the like, carbonates and bicarbonates such as sodium carbonate, potassium carbonate, sodium bicarbonate, potassium bicarbonate and the like. The base to be used is normally equal or in large excess based on the acylating agent. When pyridine is used as the base, the pyridine in large excess can also be used as the solvent. The reaction is carried out at -20 to +40°C. The reaction time is normally 10 minutes to 24 hours.
(4) Production of the compound (I) wherein X is -CH₂- and Q is -CH₂COOH
   This compound (I-4) can be produced according to the Scheme 4. 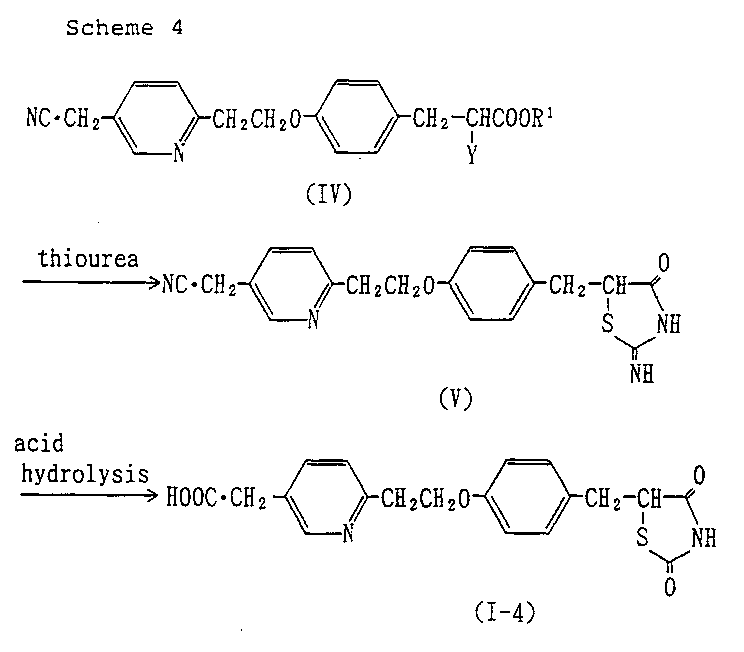 wherein Y is as defined above.
   The reaction of the compound (IV) with thiourea can be carried out according to the same manner as that described with respect to the reaction of the compound (II) with thiourea to obtain the compound (V). The acid hydrolysis of the compound (V) can be carried out according to the same manner as that described with respect to the hydrolysis of the compound (III).
(5) Production of the compound (I) wherein X is -CO- and Q is CH₃CH₂-
   This compound (I-5) can be produced according to the Scheme 5.

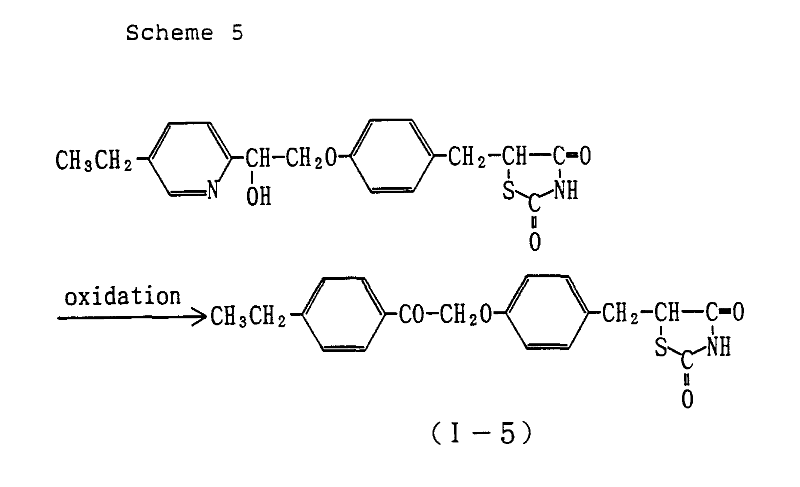

The oxidation can be carried out according to the same manner as that described with respect to the oxidation of the compound (I-1) to the compound (I-2).

The compound (I), its salt or stereoisomeric form thus obtained can be isolated and purified by known separation and purification techniques such as concentration, concentration under reduced pressure, crystallization, recrystallization, dissolution in a different solvent, chromatography or the like.

The starting material (II) can be produced according to the Scheme 6. 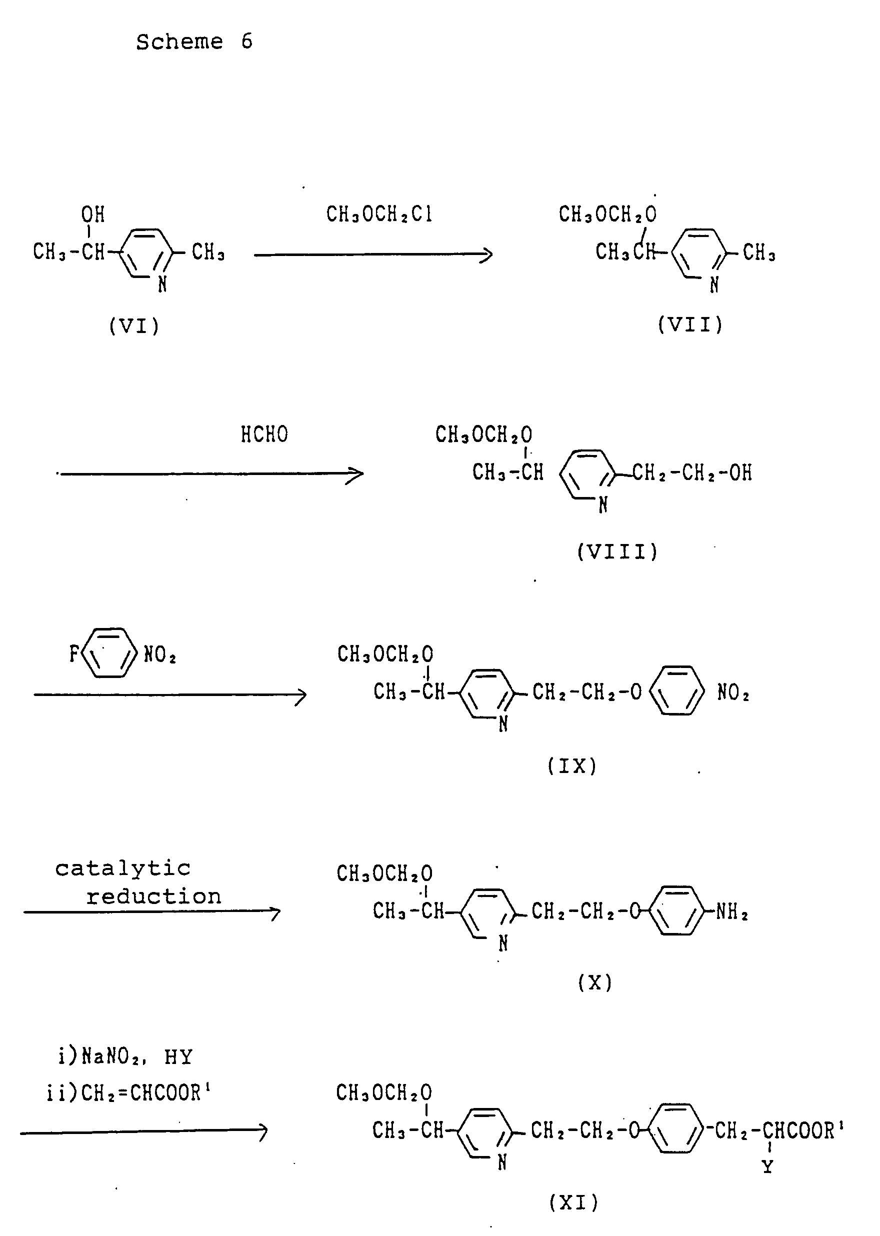 wherein Y is as defined above.

Conversion of the compound (VI) into the compound (VII) is carried out by reacting the compound (VI) with chloromethyl methyl ether in the presence of sodium hydride, sodium hydroxide, potassium hydroxide or the like. The reaction can be carried out in a solvent such as dimethylformamide, dimethyl sulfoxide, tetrahydrofuran or the like at -20 to +60°C. Then, the compound (VII) is reacted with formalin at 100 to 180°C to produce the compound (VIII). Conversion of the compound (VIII) into the compound (IX) is carried out by reacting the compound (VIII) with 4-fluoronitrobenzene in the presence of sodium hydride, sodium hydroxide, potassium hydroxide or the like. The reaction can be carried out in a solvent such as dimethylformamide, dimethyl sulfoxide, tetrahydrofuran or the like at -20 to +60°C. Catalytic hydrogenation of the compound (IX) is carried out, for example, by using palladium on carbon as a catalyst according to a conventional manner to obtain the compound (X). Conversion of the compound (X) into the compound (XI) is carried out by subjecting the compound (X) to diazotization in the presence of a hydrogen halide followed by so-called Meerwein arylation wherein the diazo compound is reacted with acrylic acid or its esters in the presence of a copper catalyst (e.g., cuprous oxide, cupric oxide, cuprous chloride, cupric chloride, cuprous bromide, cupric bromide, etc.).

The compound (IV) can be produced according to the Scheme 7.

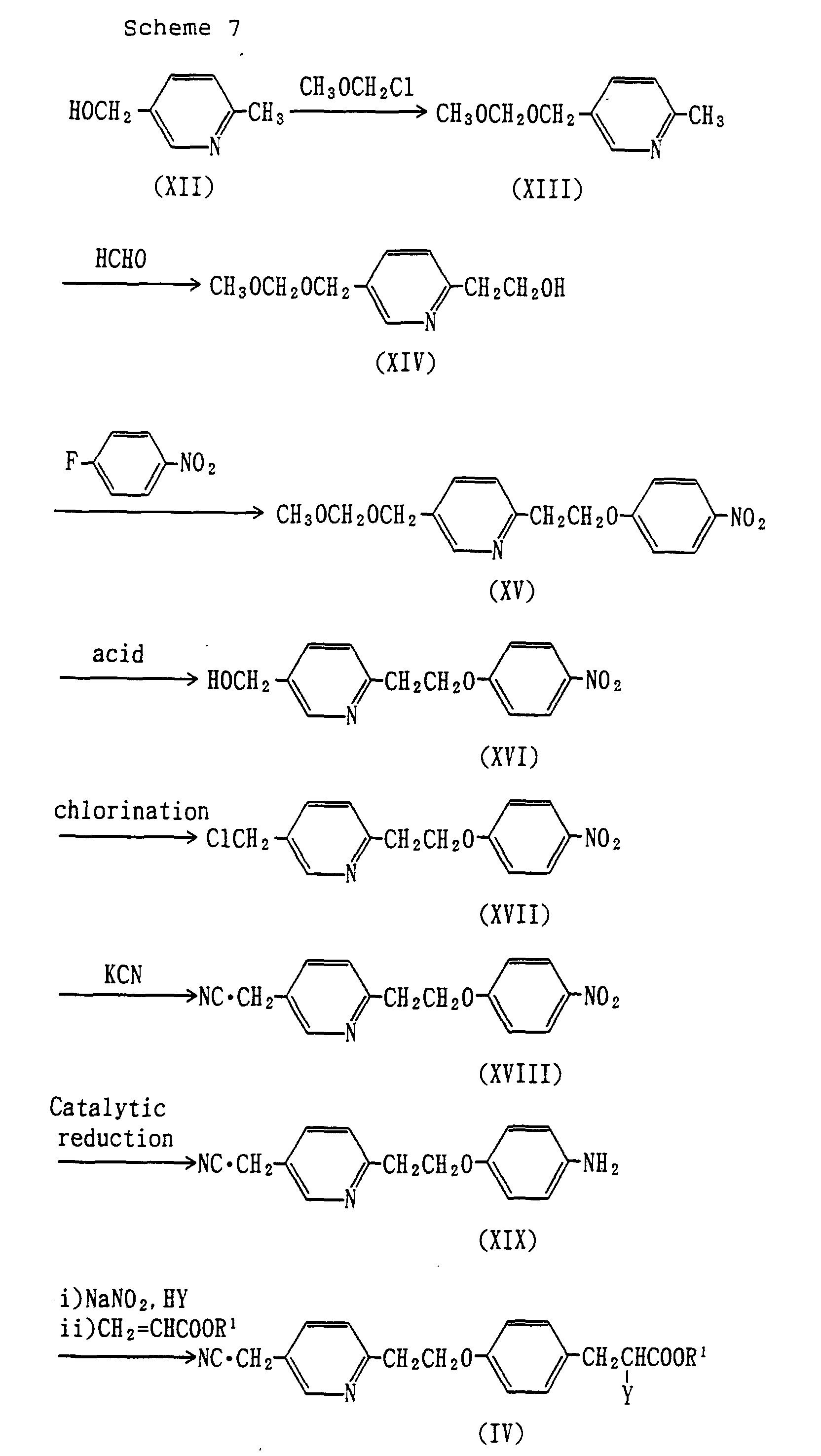

In this process, the compound (XV) prepared according to the same manner as that described with respect to the production of compound (IX) is reacted with an acid to obtain the compound (XVI). This reaction can be carried out in the presence of an acid such as hydrochloric acid, sulfuric acid or the like in a solvent containing water at 0 to 100°C. The compound (XVI) is chlorinated with thionyl chloride to obtain the compound (XVII). The compound (XVII) is reacted with potassium cyanide (or sodium cyanide) in a solvent such as acetone, dioxane, N,N-dimethylformamide, dimethylsulfoxide or the like at 0 to 100°C to obtain the compound (XVIII). The compound (XVIII) is subjected to catalytic reduction according to the same manner as that described with respect to the compound (IX) to obtain the compound (IV).

The starting material of the above Scheme 5 is known and disclosed in U.S. Patent No. 4,582,839.

The following Experiment shows that the compound (I) of the present invention has excellent hypoglycemic and hypolipidemic activities.
Experiment
Hypoglycemic and hypolipidemic activities
in mice

Test compounds were mixed with laboratory chow diet (CE-2, Clea Japan Inc., Tokyo, Japan) in a proportion of 0.005% by weight. The dietary admixture was given freely to KKA^{y}-mice (male, 9 to 10 weeks old) for 4 days. During the period, water was given freely. Blood was collected from the orbitral venous plexus. The blood sugar level was determined according to glucose oxidase method. The plasma lipid level was enzymatically determined by using Kit Cleantech TG-S (Iatron, Tokyo, Japan). The results are shown in Table 1. Values represent % reduction from control groups.

**Table 1**

| Compound (Example No.) | Hypoglycemic activity (%) | Hypolipidemic activity (%) |
|---|---|---|
| 1 | 46 | 5 |
| 2 | 56 | 43 |

As is clear from Table 1, the compound (I) of the present invention has excellent hypoglycemic and hypolipidemic activities, and is useful for a therapeutic agent for treating diabetes, hyperlipidemia and the like.

Further, the compound (I) of the present invention causes neither lactic acidosis nor serious hypoglycemia.

As described hereinabove, according to the present invention, there is provided novel thiazolidinedione derivatives or salts thereof which have excellent hypoglycemic and hypolipidemic activities without causing lactic acidosis and hypoglycemia.

The following Examples and Reference Examples further illustrates the present invention in detail but are not to be construed to limit the scope of the invention.

### Example 1

A mixture of methyl 2-bromo-3-[4-[2-[5-(1-methoxymethoxyethyl)-2-pyridyl]ethoxy]phenyl]propionate (27.0 g), thiourea (4.6 g), sodium acetate (4.9 g) and ethanol (250 ml) was heated under reflux for 4 hours. To the reaction mixture was added 2N hydrochloric acid (250 ml). The mixture was further heated under reflux for 20 hours and concentrated under reduced pressure. The residue was neutralized with saturated aqueous sodium bicarbonate solution and extracted with ethyl acetate. The ethyl acetate layer was washed with water, dried over anhydrous magnesium sulfate and concentrated under reduced pressure. The oily residue thus obtained was subjected to silica gel column chromatography. A fraction eluted with chloroform-methanol (40:1, v/v) gave 5-[4-[2-[5-(l-hydroxyethyl)-2-pyridyl]ethoxy]benzyl]-2,4-thiazolidinedione (14.8 g, yield: 66%). This crude compound was recrystallized from ethanol to obtain colorless prisms, m.p. 155-156°C.

| Elemental Analysis for C₁₉H₂₀N₂O₄S·1/4C₂H₅OH, | | | |
|---|---|---|---|
| Calcd. | C, 61.00; | H, 5.64; | N, 7.30 |
| Found | C, 60.87; | H, 5.70; | N, 7.31 |

### Example 2

Acetic anhydride (25 ml) was added to a solution of 5-[4-[2-[5-(1-hydroxyethyl)-2-pyridyl]ethoxy]benzyl]-2,4-thiazolidinedione (8.7 g) in dimethyl sulfoxide (100 ml). The mixture was allowed to stand at room temperature for 4 days. The reaction mixture was poured into water and extracted with ethyl acetate. The ethyl acetate layer was washed with water, dried over anhydrous magnesium sulfate and concentrated under reduced pressure. The oily residue was subjected to silica gel column chromatography. A fraction eluted with chloroform-methanol (100:1, v/v) gave 5-[4-[2-(5-acetyl-2-pyridyl)ethoxy]benzyl]-2,4-thiazolidinedione. This compound was recrystallized from ethanol to obtain colorless prisms, m.p. 114-115°C.

| Elemental Analysis for C₁₉H₁₈N₂O₄S·1/4C₂H₅OH, | | | |
|---|---|---|---|
| Calcd. | C, 61.32; | H, 5.15; | N, 7.33 |
| Found | C, 61.40; | H, 5.08; | N, 7.43 |

### Example 3

5-[4-[2-[5-(1-Hydroxyethyl)-2-pyridyl]ethoxy]-benzyl]-2,4-thiazolidinedione (0.3 g) was dissolved in hydrogen chloride-ethanol (25%, 1 ml). The solution was stirred at room temperature for 30 minutes and precipitated crystals were filtered off. The crystals were recrystallized from ethanol to obtain 5-[4-[2-[5-(1-hydroxyethyl)-2-pyridyl]ethoxy]benzyl]-2,4-thiazolidinedione hydrochloride (0.21 g, yield: 62 %) as colorless prisms, m.p. 212-213°C.

| Elemental Analysis for C₁₉H₂₀N₂O₄S·HCl ·1/4 C₂H₅OH, | | | |
|---|---|---|---|
| Calcd. | C, 55.71; | H, 5.39; | N, 6.66 |
| Found | C, 55.75; | H, 5.36; | N, 6.77 |

### Example 4

A mixture of 5-[4-[2-[5-(1-hydroxyethyl)-2-pyridyl]benzyl]-2,4-thiazolidinedione·1/4 ethanol (384 mg), acetic anhydride (0.5 ml) and pyridine (5 ml) was stirred at room temperature for 12 hours. After addition of water (5 ml), the mixture was concentrated under reduced pressure and the remaining crystals were filtered off. The crystals were recrystallized from ethyl acetate-hexane to obtain 5-[4-[2-[5-(1-acetoxyethyl)-2-pyridyl]ethoxy]benzyl]-2,4-thiazolidinedione (345 mg, yield: 83%) as colorless prisms, m.p. 143-144°C.

| Elemental Analysis for C₂₁H₂₂N₂O₅S, | | | |
|---|---|---|---|
| Calcd. | C, 60.85; | H, 5.35; | N, 6.76 |
| Found | C, 60.73; | H, 5.45; | N, 6.79 |

### Example 5

A mixture of 5-[4-[2-(5-cyanomethyl-2-pyridyl)ethoxy]benzyl]-2-imino-4-thiazolidinone (2.3 g) and 4N hydrochloric acid (100 ml) was heated under reflux for 24 hours and concentrated under reduced pressure. The residue was neutralized with saturated aqueous solution of sodium bicarbonate and acidified with addition of acetic acid. Crystals precipitated were filtered off and dissolved in ethyl acetate (200 ml)-methanol (200 ml). The resulting solution was washed with water and dried over magnesium sulfate and the solvent was distilled off to obtain 5-[4-[2-(5-carboxymethyl-2-pyridyl)ethoxy]benzyl]-2,4-thiazolidinedione (1.9 g, yield: 79%). This compound was recrystallized from ethanol to obtain colorless prisms, m.p. 144-145°C.

| Elemental Analysis for C₁₉H₁₈N₂O₅S, | | | |
|---|---|---|---|
| Calcd. | C, 59.06, | H, 4.70; | N, 7.25 |
| Found | C, 58.89; | H, 4.69; | N, 7.08 |

### Example 6

A mixture of 5-[4-[2-(5-ethyl-2-pyridyl)-2-hydroxyethoxy]benzyl]-2,4-thiazolidinedione·1/2 C₂H₅OH (396 mg), acetic anhydride (1.5 ml) and dimethylsulfoxide (4 ml) was stirred at room temperature for 5 hours and then poured into water. The mixture was extracted with ethyl acetate. The ethyl acetate layer was washed with water and dried over magnesium sulfate and the solvent was distilled off. The oily residue was subjected to silica gel chromatography. A fraction eluted with chloroform-methanol (100:1, v/v) gave 5-[4-[2-(5-ethyl-2-pyridyl)-2-oxoethoxy]benzyl]-2,4-thiazolidinedione (75 mg, yield: 20%). This was recrystallized from ethyl acetate-hexane to obtain colorless prisms, m.p. 148-149°C.

| Elemental Analysis for C₁₉H₁₈N₂O₄S, | | | |
|---|---|---|---|
| Calcd. | C, 61.61; | H, 4.90; | N, 7.56 |
| Found | C, 61.39; | H, 4.91; | N, 7.37 |

### Example 7

### Preparation of tablets

Tablets are prepared according to the following formulation.

| | | |
|---|---|---|
| (1) | 5-[4-[2-[5-(1-hydroxyethyl)-2-pyridyl]ethoxy]benzyl]-2,4-thiazolidinedione 1/4 ethanol solvate | 100 g |
| (2) | Lactose | 50 g |
| (3) | Corn starch | 15 g |
| (4) | Calcium carboxymethylcellulose | 44 g |
| (5) | Magnesium stearate | 1 g |
| | 1000 tablets | 210 g |

All the ingredients of (1), (2) and (3), and 30 g of the ingredient (4) were kneaded with water. The mixture was dried under vacuum and then granulated. The granules were mixed with 14 g of the ingredient (4) and 1 g of the ingredient (5). The resulting mixture was compressed into tablets by a tablet machine to produce 1000 tablets of 8 mm in diameter containing 100 mg of the ingredient (1) per tablet.

### Reference Example 1

Sodium hydride in oil (60%, 7.0 g) was added with ice-cooling to a solution of 5-(1-hydroxyethyl)-2-methylpyridine (20.0 g) in N,N-dimethylformamide (120 ml). The mixture was stirred for 15 minutes. Then, chloromethyl methyl ether (14.1 g) was added dropwise at the same temperature. The reaction mixture was further stirred for 30 minutes with ice-cooling. The mixture was poured into water and extracted with ethyl acetate. The ethyl acetate layer was washed with water, dried over anhydrous magnesium sulfate and subjected to distillation under reduced pressure to obtain 5-(1-methoxymethoxyethyl)-2-methylpyridine (21.5 g, yield: 81 %), b.p. 78-80°C/0.8 mmHg.

### Reference Example 2

The mixture of 5-(1-methoxymethoxyethyl)-2-methylpyridine (21.0 g) and formalin (37% aq. HCHO, 14.1 g) was heated at 150 to 160°C for 8 hours in a sealed tube. The reaction mixture was concentrated under reduced pressure and then the residue was subjected to silica gel column chromatography. A fraction eluted with chloroform-methanol (25:1, v/v) gave 2-[5-(1-methoxymethoxyethyl)-2-pyridyl]ethanol (7.8 g, yield: 32 %) as oil.

NMR (δ ppm in CDCl₃): 1.49 (3H, d, J=7Hz), 3.01 (2H, t, J=6Hz), 3.36 (3H, s), 4.03 (2H, t, J=6Hz), 4.53 (1H, d, J=7Hz), 4.61 (1H, d, J=7Hz), 4.77 (1H, d, J=7Hz), 7.15 (1H, d, J=8Hz), 7.62 (1H, dd, J=8 and 2Hz), 8.46 (1H, d, J=2Hz).

### Reference Example 3

Sodium hydride in oil (60%, 1.6 g) was added in small portions with ice-cooling to a solution of 2-[5-(1-methoxymethoxyethyl)-2-pyridyl]ethanol (7.5 g) and 4-fluoronitrobenzene (5.0 g) in N,N-dimethylformamide (50 ml). The reaction mixture was stirred with ice-cooling for additional 1 hour, poured into water and extracted with ethyl acetate. The ethyl acetate layer was washed with water, dried over anhydrous magnesium sulfate and concentrated under reduced pressure. The oily residue was subjected to silica gel column chromatography. A fraction eluted with chloroform-methanol (40:1, v/v) gave 5-(1-methoxymethoxyethyl)-2-[2-(4-nitrophenoxy)ethyl]pyridine (8.4 g, yield: 71 %) as oil.

NMR (δ ppm in CDCl₃): 1.49 (3H, d, J=7Hz), 3.30 (2H, t, J=6Hz), 3.36 (3H, s), 4.48 (2H, t, J=6Hz), 4.52 (1H, d, J=7Hz), 4.62 (1H, d, J=7Hz), 4.79 (1H, d, J=7Hz), 6.97 (2H, d, J=9Hz), 7.25 (1H, d, J=8Hz), 7.64 (1H, dd, J=8 and 2 Hz), 8.18 (2H, d, J=9Hz), 8.53 (1H, d, J=2Hz).

### Reference Example 4

A mixture of 5-(1-methoxymethoxyethyl)-2-[2-(4-nitrophenoxy)ethyl]pyridine (8.2 g), 5% palladium-carbon (50% wet, 0.8 g) and ethyl acetate (100 ml) was subjected to catalytic reduction at room temperature and at the pressure of 1 atm. The catalyst was filtered off and the filtrate was concentrated under reduced pressure. The oily residue was dissolved in acetone (80 ml)-hydrobromic acid (47%, 16.9 g). A solution of sodium nitrite (1.9 g) in water (5 ml) was added dropwise at 0 to 5°C. The reaction mixture was stirred at 5°C for additional 20 minutes. Then, methyl acrylate (12.7 g) was added thereto and the resulting mixture was warmed to 37°C. While this mixture was stirred vigorously, cuprous oxide (0.3 g) was added in small portions to the mixture. After the evolution of nitrogen gas had ceased, the reaction mixture was concentrated under reduced pressure. The residue was made basic with conc. NH₄OH and extracted with ethyl acetate. The ethyl acetate layer was washed with water, dried over anhydrous magnesium sulfate and concentrated under reduced pressure. The oily residue was subjected to silica gel column chromatography. A fraction eluted with ether-hexane-triethylamine (25:25:1, v/v) gave methyl 2-bromo-3-[4-[2-[5-(1-methoxymethoxyethyl)-2-pyridyl]ethoxy]phenyl]propionate (5.6 g, yield: 50 %) as oil.

NMR (δ ppm in CDCl₃): 1.48 (3H, d, J=7Hz), 3.1-3.4 (2H, m), 3.24 (2H, t, J=6Hz), 3.36 (3H, s), 3.71 (3H, s), 4.33 (2H, t, J=6Hz), 4.53 (1H, d, J=7Hz), 4.61 (1H, d, J=7Hz), 4.5-4.7 (1H, m), 4.77 (1H, q, J=6Hz), 6.83 (2H, d, J=9Hz), 7.09 (2H, d, J=9Hz), 7,245 (1H, d, J=8Hz), 7.61 (1H, dd, J=8 and 2 Hz), 8.50 (1H, d, J=2Hz).

### Reference Example 5

A solution of methyl 6-methylnicotinate (45.4 g) in tetrahydrofuran (50 ml) was added dropwise to a suspension of lithium aluminium hydride (LiAlH₄, 5.7 g) in tetrahydrofuran (250 ml) at room temperature. The mixture was stirred at room temperature for additional 1 hour and water (30 ml) was added dropwise with ice-cooling. Insoluble materials were filtered off and the filtrate was concentrated. The residue was distilled under reduced pressure to obtain 6-methyl-3-pyridylmethanol (31 g, yield: 84 %), b.p. 98-100°C/0.5 mmHg.

### Reference Example 6

6-Methyl-3-pyridylmethaol (30.8 g) was dissolved in tetrahydrofuran (200 ml) and sodium hydride in oil (60%, 11 g) was added thereto. The mixture was stirred with ice-cooling for 30 minutes. Then, chloromethyl methyl ether (22.1 g) was added dropwise with ice-cooling and the mixture was stirred for additional 1 hour. The mixture was poured into water and extracted with ethyl acetate. The ethyl acetate layer was washed with water and dried over magnesium sulfate and the solvent was distilled off under reduced pressure to obtain 5-methoxymethoxymethyl-2-methylpyridine (31 g, yield: 74%), b.p. 85-87°C/0.5 mmHg.

### Reference Example 7

A mixture of 5-methoxymethoxymethyl-2-methylpyridine (30.5 g) and formalin (37% aq., HCHO, 22.2 g) was heated in a sealed tube at 150 to 160°C for 8 hours. The reaction mixture was concentrated under reduced pressure and the oily residue was subjected to silica gel column chromatography. A fraction eluted with chloroform-methanol (20:1, v/v) gave 2-(5-methoxymethoxymethyl-2-pyridyl)ethanol (11.8 g, yield: 33%) as oil.

NMR (δ ppm in CDCl₃): 3.02 (2H, t, J=6Hz), 3.41 (3H, s), 4.02 (2H, t, J=6Hz), 4.59 (2H, s), 4.71 (2H, s), 7.16 (1H, d, J=8Hz), 7.64 (1H, dd, J=8 and 2Hz), 8.48 (1H, d, J=2Hz).

### Reference Example 8

To a solution of 2-(5-methoxymethoxymethyl-2-pyridyl)ethanol (11.6 g) and 4-fluoronitrobenzene (8.5 g) in N,N-dimethylformamide (100 ml) was added by portions sodium hydride in oil (60%, 2.8 g) with ice-cooling. The reaction mixture was stirred with ice-cooling for additional 1 hour, poured into water and then extracted with ethyl acetate. The ethyl acetate layer was washed with water, dried over magnesium sulfate and concentrated under reduced pressure. The oily residue was subjected to silica gel column chromatography. A fraction eluted with chloroform-methanol (40:1, v/v) gave 5-methoxymethoxymethyl-2-[2-(4-nitrophenoxy)ethyl]pyridine (14.7 g, yield: 79%) as oil.

NMR (δ ppm in CDCl₃): 3.30 (2H, t, J=6Hz), 3.41 (3H, s), 4.47 (2H, t, J=6Hz), 4.60 (2H, s), 4.71 (2H, s), 6.94 (2H, d, J=9Hz), 7.26 (1H, d, J=8Hz), 7.66 (1H, dd, J=8 and 2Hz), 8.17 (2H, d, J=9Hz), 8.54 (1H, d, J=2Hz).

### Reference Example 9

A mixture of 5-methoxymethoxymethyl-2-[2-(4-nitrophenoxy)ethyl]pyridine (14.5 g), 2N hydrochloric acid (50 ml) and methanol (50 ml) was stirred under reflux for 2 hours. The reaction mixture was concentrated and the residue was neutralized with saturated aqueous solution of sodium bicarbonate and extracted with ethyl acetate. The ethyl acetate layer was washed with water, dried over magnesium sulfate and concentrated under reduced pressure to obtain 5-hydroxymethyl-2-[2-(4-nitrophenoxy)ethyl]pyridine (9.7 g, yield: 88%). This compound was recrystallized from ethyl acetate to obtain colorless prisms, m.p. 144-145°C.

| Elemental Analysis for C₁₄H₁₄N₂O₄, | | | |
|---|---|---|---|
| Calcd. | C, 61.31; | H, 5.14; | N, 10.21 |
| Found | C, 61.01; | H, 5.14; | N, 10.13. |

### Reference Example 10

To a solution of 5-hydroxymethyl-2-[2-(4-nitrophenoxy)ethyl]pyridine (9.4 g) in chloroform (100 ml) was added thionyl chloride (7.0 g) and the mixture was stirred under reflux for 30 minutes. After cooling, the reaction mixture was washed with saturated aqueous solution of sodium bicarbonate and then water, dried over magnesium sulfate and concentrated under reduced pressure to obtain 5-chloromethyl-2-[2-(4-nitrophenoxy)ethyl]pyridine (9.7 g, yield: 85%). The compound was recrystallized from ethyl acetate-hexane to obtain colorless needles, m.p. 77-78°C.

| Elemental Analysis for C₁₄H₁₃N₂O₃Cl, | | | |
|---|---|---|---|
| Calcd. | C, 57.44; | H, 4.48; | N, 9.57 |
| Found | C, 57.07, | H, 4.42; | N, 9.46. |

### Reference Example 11

A mixture of 5-chloromethyl-2-[2-(4-nitrophenoxy)-ethyl]pyridine (9.5 g), potassium cyanide (3.2 g) and N,N-dimethylformamide (100 ml) was stirred at 60°C for 2 hours. The reaction mixture was poured into water and extracted with ethyl acetate. The ethyl acetate layer was washed with water, dried over magnesium sulfate and concentrated under reduced pressure to obtain 5-cyanomethyl-2-[2-(4-nitrophenoxy)ethyl]pyridine (9.0 g, yield: 98%). This compound was recrystallized from ethyl acetate-hexane to obtain colorless prisms, m.p. 94-95°C.

| Elemental Analysis for C₁₅H₁₃N₃O₃, | | | |
|---|---|---|---|
| Calcd. | C, 63.60; | H, 4.63; | N, 14.83 |
| Found | C, 63.19; | H, 4.63; | N, 14.41. |

### Reference Example 12

A mixture of 5-cyanomethyl-2-[2-(4-nitrophenoxy)ethyl]pyridine (8.8 g), 5% palladium-carbon (50% wet, 1.0 g) and ethyl acetate (100 ml) was subjected to catalytic reduction at room temperature and at the pressure of 1 atm. The catalyst was filtered off and the filtrate was concentrated under reduced pressure. The oily residue was dissolved in acetone (80 ml)-hydrobromic acid (47%, 21.4 g). To the solution was added dropwise a solution of sodium nitrite (2.4 g) in water (5 ml) at 0 to 5°C. The reaction mixture was stirred at 5°C for additional 20 minutes and methyl acrylate (16.1 g) was added thereto. The mixture was warmed to 37°C and cuprous oxide (0.3 g) was added in small portions, while the mixture was vigorously stirred. After the evolution of nitrogen gas had ceased, the reaction mixture was concentrated under reduced pressure and the residue was made basic with conc. NH₄OH and extracted with ethyl acetate. The ethyl acetate layer was washed with water, dried over magnesium sulfate, and concentrated under reduced pressure. The oily residue was subjected to silica gel column chromatography. A fraction eluted with ether-hexane-triethylamine (25:25:1, v/v) gave methyl 2-bromo-3-[4-[2-(5-cyanomethyl-2-pyridyl)ethoxy]-phenyl]propionate (7.3 g, yield: 58%) as oil.

NMR (δ ppm, CDCl₃): 3.1-3.4 (2H, m), 3.26 (2H, t, J=6Hz), 3.71 (2H, s), 3.74 (3H, s), 4.3-4.4 (1H, m), 4.33 (2H, t, J=6Hz), 6.76 (2H, d, J=9Hz), 7.2-7.4 (3H, m), 7.65 (1H, dd, J=8 and 2Hz), 8.50 (1H, d, J=2Hz).

### Reference Example 13

A mixture of methyl 2-bromo-3-[4-[2-(5-cyanomethyl-2-pyridyl)ethoxy]phenyl]propionate (7.2 g), thiourea (1.2 g), sodium acetate (1.3 g) and ethanol (80 ml) was heated under reflux for 5 hours and concentrated under reduced pressure. To the residue was added saturated aqueous sodium bicarbonate solution (100 ml)-ether (50 ml) and the mixture was stirred for 15 minutes. The precipitated crystals were filtered off to obtain 5-[4-[2-(5-cyanomethyl-2-pyridyl)ethoxy]benzyl]-2-imino-4-thiazolidinone (2.5 g, yield: 38%). This compound was recrystallized from chloroform-methanol to obtain colorless prisms, m.p. 211-212°C.

| Elemental Analysis for C₁₉H₁₈N₄O₂S, | | | |
|---|---|---|---|
| Calcd. | C, 62.28; | H, 4.95; | N, 15.29 |
| Found | C, 62.21; | H, 4.89; | N, 15.15. |

## Claims

1. A thiazolidinedione derivative of the general formula (I): 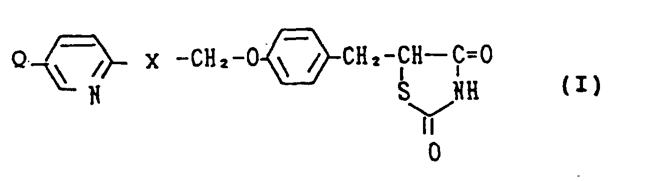 wherein X is -CO- and Q is CH₃CH₂-, or X is -CH₂- and Q is CH₃CO-, CH₃CH(OR)- or -CH₂COOH, wherein R is H or an acyl group selected from formyl, C₂₋₆ alkylcarbonyl, C₈₋₉ aralkylcarbonyl and C₇₋₈ arylcarbonyl, wherein the aralkyl or aryl group optionally has one or more substituents selected from halogen, C₁₋₄ alkoxy and CF₃; or a pharmaceutically-acceptable salt thereof.

2. A thiazolidinedione derivative according to claim 1, wherein X is -CH₂- and Q is CH₃CO- or CH₃CH(Oacyl)-, or X is -CO- and Q is CH₃CH₂-.

3. A thiazolidinedione derivative according to claim 1, wherein X is -CH₂- and Q is CH₃CH(OH)- or -CH₂COOH.

4. The thiazolidinedione derivative according to claim 1, which is 5-[4-[2-(5-acetyl-2-pyridyl)ethoxy]benzyl]-2,4-thiazolidinedione or its salt.

5. The thiazolidinedione derivative according to claim 1, which is 5-[4-[2-[5-(1-hydroxyethyl)-2-pyridyl]ethoxy]benzyl]-2,4-thiazolidinedione or its salt.

6. The thiazolidinedione derivative according to claim 1 which is 5-[4-[2-[5-(1-acetoxyethyl)-2-pyridyl]ethoxy]benzyl]-2,4-thiazolidinedione or its salt.

7. The thiazolidinedione derivative according to claim 1 which is 5-[4-[2-[5-(1-propionyloxyethyl)-2-pyridyl]ethoxy]benzyl]-2,4-thiazolidinedione or its salt.

8. The thiazolidinedione derivative according to claim 1 which is 5-[4-[2-[5-(1-butyryloxyethyl)-2-pyridyl]ethoxy]benzyl]-2,4-thiazolidinedione or its salt.

9. The thiazolidinedione derivative according to claim 1 which is 5-[4-[2-[5-(1-isobutyryloxyethyl)-2-pyridyl]ethoxy]benzyl]-2,4-thiazolidinedione or its salt.

10. The thiazolidinedione derivative according to claim 1 which is 5-[4-[2-[5-(1-valeryloxyethyl)-2-pyridyl]ethoxy]benzyl]-2,4-thiazolidinedione or its salt.

11. The thiazolidinedione derivative according to claim 1 which is 5-[4-[2-[5-(1-isovaleryloxyethyl)-2-pyridyl]ethoxy]benzyl]-2,4-thiazolidinedione or its salt.

12. The thiazolidinedione derivative according to claim 1 which is 5-[4-[2-[5-(1-pivaloyloxyethyl)-2-pyridyl]ethoxy]benzyl]-2,4-thiazolidinedione or its salt.

13. The thiazolidinedione derivative according to claim 1 which is 5-[4-[2-[5-(1-benzoyloxyethyl)-2-pyridyl]ethoxy]benzyl]-2,4-thiazolidinedione or its salt.

14. The thiazolidinedione derivative according to claim 1 which is 5-[4-[2-(5-carboxymethyl-2-pyridyl]ethoxy]benzyl]-2,4-thiazolidinedione or its salt.

15. The thiazolidinedione derivative according to claim 1, which is 5-[4-[2-(5-ethyl-2-pyridyl)-2-oxoethoxy]benzyl]-2,4-thiazolidinedione or its salt.

16. A thiazolidinedione derivative according to any preceding claim, in the form of a pure stereoisomer.

17. A process for producing a thiazolidinedione derivative according to claim 1, which comprises:
(a) for the production of the derivative of the general formula (I) wherein X is -CH₂- and Q is CH₃CH(OH)-, reacting a compound of the formula (II) 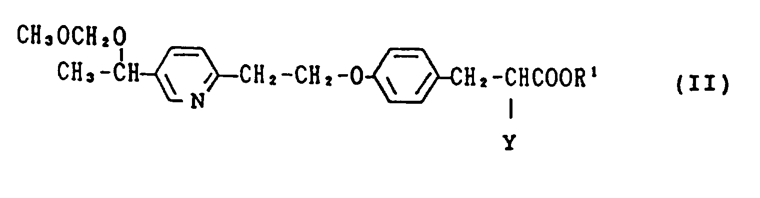 wherein R¹ is a hydrogen atom or lower alkyl, and Y is a leaving group, with thiourea and then subjecting the resulting compound of the formula (III) 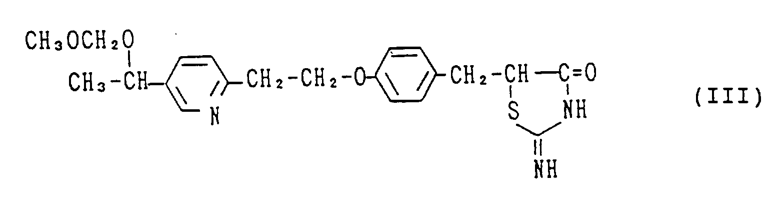 to acid hydrolysis; or
(b) for the production of the derivative of the general formula (I) wherein X is -CH₂- and Q is CH₃CO-, oxidizing the derivative of the general formula (I) wherein Q is CH₃CH(OH)-; or
(c) for the production of the derivative of the general formula (I) wherein X is -CH₂- and Q is CH₃CH(OR)-and R is other than a hydrogen atom, acylating the derivative of the general formula (I) wherein Q is CH₃CH(OH)-; or
(d) for the production of the derivative of the general formula (I) wherein X is -CH₂- and Q is -CH₂COOH, subjecting a compound of the formula (V): 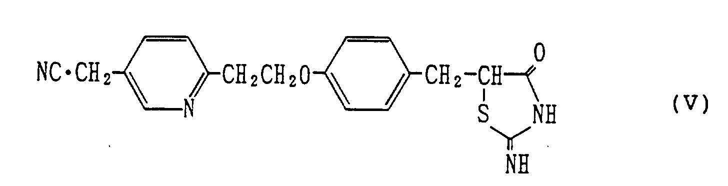 to acid hydrolysis;
(e) for the production of the derivatives of the general formula (I) wherein X is -CO- and Q is CH₃CH₂-, oxidising a compound of the formula 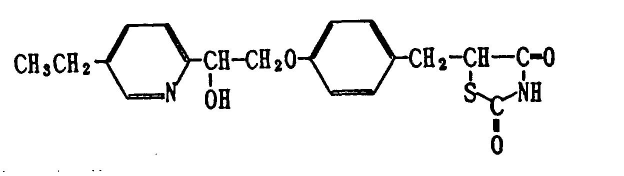 and then optionally converting the resulting derivative into its salt.

18. A process according to claim 17, wherein the thiazolidinedione derivative is according to any of claims 2 to 16.

19. A pharmaceutical composition for treating diabetes, comprising a thiazolidinedione derivative according to any of claims 1 to 16 and a pharmacologically-acceptable carrier, excipient or diluent.

20. Use of a thiazolidinedione derivative according to any of claims 1 to 16, for the manufacture of a medicament for use in treating diabetes.

## Patentansprüche

1. Thiazolidindion-Derivat der allgemeinen Formel (I): 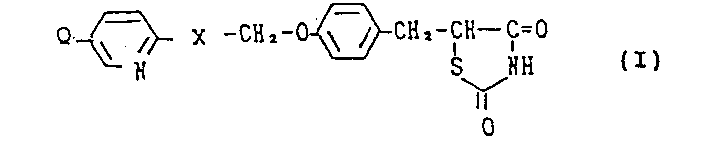 worin X -CO- ist und Q CH₃CH₂- ist, oder X -CH₂- ist und Q CH₃CO-, CH₃CH(OR)-oder -CH₂COOH ist, worin R H oder eine aus Formyl, C₂₋₆-Alkylcarbonyl, C₈₋₉-Aralkylcarbonyl und C₇₋₈-Arylcarbonyl gewählte Acylgruppe ist, worin die Aralkyl-oder Arylgruppe gegebenenfalls einen oder mehrere von Halogen, C₁₋₄-Alkoxy und CF₃ gewählte Substituenten aufweist; oder ein pharmazeutisch annehmbares Salz davon.

2. Thiazolidindion-Derivat gemäß Anspruch 1, worin X -CH₂- ist und Q CH₃CO- oder CH₃CH(O-acyl)- ist, oder X -CO- ist und Q CH₃CH₂- ist.

3. Thiazolidindion-Derivat gemäß Anspruch 1, worin X -CH₂- ist und Q CH₃CH(OH)-oder -CH₂COOH ist.

4. Thiazolidindion-Derivat gemäß Anspruch 1, welches 5-[4-[2-(5-Acetyl-2-pyridyl)-ethoxy]benzyl]-2,4-thiazolidindion oder dessen Salz ist.

5. Thiazolidindion-Derivat gemäß Anspruch 1, welches 5-[4-[2-[5-(1-Hydroxyethyl)-2-pyridyl]ethoxy]benzyl]-2,4-thiazolidindion oder dessen Salz ist.

6. Thiazolidindion-Derivat gemäß Anspruch 1, welches 5-[4-[2-[5-(1-Acetoxyethyl)-2-pyridyl]ethoxy]benzyl]-2,4-thiazolidindion oder dessen Salz ist.

7. Thiazolidindion-Derivat gemäß Anspruch 1, welches 5-[4-[2-[5-(1-Propionyloxyethyl)-2-pyridyl]ethoxy]benzyl]-2,4-thiazolidindion oder dessen Salz ist.

8. Thiazolidindion-Derivat gemäß Anspruch 1, welches 5-[4-[2-[5-(1-Butyryloxyethyl)-2-pyridyl]ethoxy]benzyl]-2,4-thiazolidindion oder dessen Salz ist.

9. Thiazolidindion-Derivat gemäß Anspruch 1, welches 5-[4-[2-[5-(1-Isobutyryloxyethyl)-2-pyridyl]ethoxy]benzyl]-2,4-thiazolidindion oder dessen Salz ist.

10. Thiazolidindion-Derivat gemäß Anspruch 1, welches 5-[4-[2-[5-(1-Valeryloxyethyl)-2-pyridyl]ethoxy]benzyl]-2,4-thiazolidindion oder dessen Salz ist.

11. Thiazolidindion-Derivat gemäß Anspruch 1, welches 5-[4-[2-[5-(1-Isovaleryloxyethyl)-2-pyridyl]ethoxy]benzyl]-2,4-thiazolidindion oder dessen Salz ist.

12. Thiazolidindion-Derivat gemäß Anspruch 1, welches 5-[4-[2-[5-(1-Pivaloyloxyethyl)-2-pyridyl]ethoxy]benzyl]-2,4-thiazolidindion oder dessen Salz ist.

13. Thiazolidindion-Derivat gemäß Anspruch 1, welches 5-[4-[2-[5-(1-Benzoyloxyethyl)-2-pyridyl]ethoxy]benzyl]-2,4-thiazolidindion oder dessen Salz ist.

14. Thiazolidindion-Derivat gemäß Anspruch 1, welches 5-[4-[2-(5-Carboxymethyl-2-pyridyl]ethoxy]benzyl]-2,4-thiazolidindion oder dessen Salz ist.

15. Thiazolidindion-Derivat gemäß Anspruch 1, welches 5-[4-[2-(5-Ethyl-2-pyridyl)-2-oxoethoxy]benzyl]-2,4-thiazolidindion oder dessen Salz ist.

16. Thiazolidindion-Derivat gemäß mindestens einem der vorhergehenden Ansprüche in Form eines reinen Stereosiomeren.

17. Verfahren zur Herstellung eines Thiazolidindion-Derivats gemäß Anspruch 1, umfassend:
(a) zur Herstellung des Derivats der allgemeinen Formel (I), worin X -CH₂- ist und Q CH₃CH(OH)- ist, das Umsetzen einer Verbindung der Formel (II) 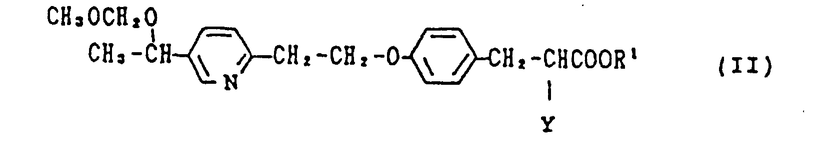 worin R¹ ein Wasserstoffatom oder ein Niederalkyl ist und Y eine Abgangsgruppe ist, mit Thioharnstoff und anschließendes Unterziehen der resultierenden Verbindung der Formel (III) 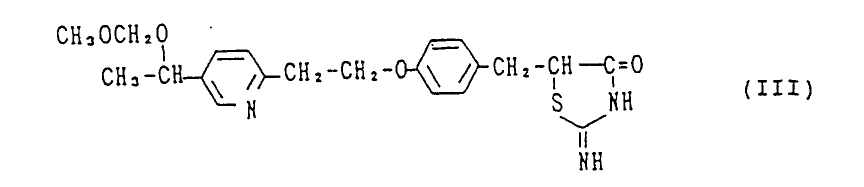 einer Säurehydrolyse; oder
(b) zur Herstellung des Derivats der allgemeinen Formel (I), worin X -CH₂- ist und Q CH₃CO- ist, das Oxidieren des Derivats der allgemeinen Formel (I), worin Q CH₃CH(OH)- ist; oder
(c) zur Herstellung des Derivats der allgemeinen Formel (I), worin X -CH₂- ist und Q CH₃CH(OR)- ist und R etwas anderes als ein Wasserstoffatom bedeutet, das Acylieren des Derivats der allgemeinen Formel (I), worin Q CH₃CH(OH)- ist; oder
(d) zur Herstellung des Derivats der allgemeinen Formel (I), worin X -CH₂- ist und Q CH₂COOH ist, das Unterziehen einer Verbindung der Formel (V): 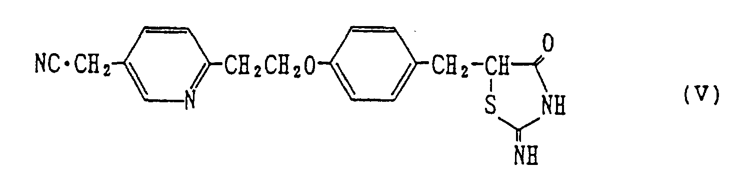 einer Säurehydrolyse;
(e) zur Herstellung der Derivate der allgemeinen Formel (I), worin X -CO- ist und Q CH₃CH₂- ist, das Oxidieren einer Verbindung der Formel 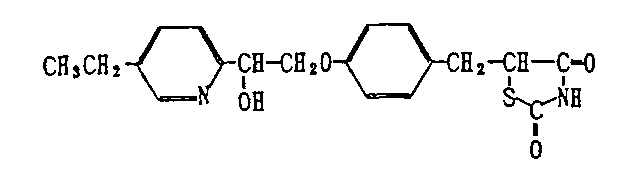 und anschließend gegebenenfalls das Umwandeln des resultierenden Derivats zu dessen Salz.

18. Verfahren gemäß Anspruch 17, bei dem das Thiazolidindion-Derivat mindestens einem der Ansprüche 2 bis 16 entspricht.

19. Pharmazeutische Zusammensetzung zur Behandlung von Diabetes, umfassend ein Thiazolidindion-Derivat gemäß mindestens einem der Ansprüche 1 bis 16 und ein(en) pharmakologisch annehmbaren(s) Träger, Vehikel oder Verdünnungsmittel.

20. Verwendung eines Thiazolidindion-Derivats gemäß mindestens einem der Ansprüche 1 bis 16 zur Herstellung eines Medikamentes zur Verwendung bei der Behandlung von Diabetes.

## Revendications

1. Dérivé de thiazolidinedione de formule générale (I) 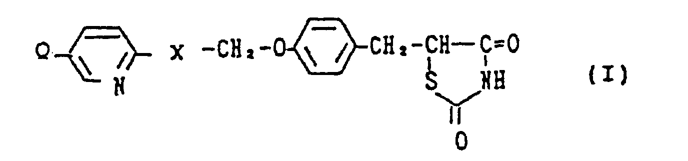 dans laquelle X est -CO- et Q est CH₃CH₂-, ou X est -CH₂- et Q est CH₃CO-, CH₃CH(OR)- ou -CH₂COOH, où R est H ou un groupe acyle choisi parmi les groupes formyle, alkylcarbonyle en C₂-C₆, aralkylcarbonyle en C₈-C₉ et arylcarbonyle en C₇-C₈, le groupe aralkyle ou aryle ayant éventuellement un ou plusieurs substituants choisis parmi les halogènes et les groupes alcoxy en C₁-C₄ et CF₃; ou un de ses sels pharmaceutiquement acceptables.

2. Dérivé de thiazolidinedione selon la revendication 1, dans lequel X est -CH₂- et Q est CH₃CO- ou CH₃CH(Oacyle)-, ou X est -CO- et Q est CH₃CH₂-.

3. Dérivé de thiazolidinedione selon la revendication 1, dans lequel X est -CH₂- et Q est CH₃CH(OH)- ou -CH₂COOH.

4. Dérivé de thiazolidinedione selon la revendication 1, qui est la 5-[4-[2-(5-acétyl-2-pyridyl)éthoxy]benzyl]-2,4-thiazolidinedione ou un de ses sels.

5. Dérivé de thiazolidinedione selon la revendication 1, qui est la 5-[4-[2-[5-(1-hydroxyéthyl)-2-pyridyl]éthoxy]benzyl]-2,4-thiazolidinedione ou un de ses sels.

6. Dérivé de thiazolidinedione selon la revendication 1, qui est la 5-[4-[2-[5-(1-acétoxyéthyl)-2-pyridyl]éthoxy]benzyl]-2,4-thiazolidinedione ou un de ses sels.

7. Dérivé de thiazolidinedione selon la revendication 1, qui est la 5-[4-[2-[5-(1-propionyloxyéthyl)-2-pyridyl]éthoxy]benzyl]-2,4-thiazolidinedione ou un de ses sels.

8. Dérivé de thiazolidinedione selon la revendication 1, qui est la 5-[4-[2-[5-(1-butyryloxyéthyl)-2-pyridyl]éthoxy]benzyl]-2,4-thiazolidinedione ou un de ses sels.

9. Dérivé de thiazolidinedione selon la revendication 1, qui est la 5-[4-[2-[5-(1-isobutyryloxyéthyl)-2-pyridyl]éthoxy]benzyl]-2,4-thiazolidinedione ou un de ses sels.

10. Dérivé de thiazolidinedione selon la revendication 1, qui est la 5-[4-[2-[5-(1-valéryloxyéthyl)-2-pyridyl]éthoxy]benzyl]-2,4-thiazolidinedione ou un de ses sels.

11. Dérivé de thiazolidinedione selon la revendication 1, qui est la 5-[4-[2-[5-(1-isovaléryloxyéthyl)-2-pyridyl]éthoxy]benzyl]-2,4-thiazolidinedione ou un de ses sels.

12. Dérivé de thiazolidinedione selon la revendication 1, qui est la 5-[4-[2-[5-(1-pivaloyloxyéthyl)-2-pyridyl]éthoxy]benzyl]-2,4-thiazolidinedione ou un de ses sels.

13. Dérivé de thiazolidinedione selon la revendication 1, qui est la 5-[4-[2-[5-(1-benzoyloxyéthyl)-2-pyridyl]éthoxy]benzyl]-2,4-thiazolidinedione ou un de ses sels.

14. Dérivé de thiazolidinedione selon la revendication 1, qui est la 5-[4-[2-(5-carboxyméthyl-2-pyridyl)éthoxy]benzyl]-2,4-thiazolidinedione ou un de ses sels.

15. Dérivé de thiazolidinedione selon la revendication 1, qui est la 5-[4-[2-(5-éthyl-2-pyridyl)-2-oxoéthoxy]benzyl]-2,4-thiazolidinedione ou un de ses sels.

16. Dérivé de thiazolidinedione selon l'une quelconque des revendications précédentes, sous forme d'un stéréoisomère pur.

17. Procédé de production d'un dérivé de thiazolidinedione selon la revendication 1, qui comprend:
(a) pour la production du dérivé de formule générale (I) dans laquelle X est -CH₂- et Q est CH₃CH(OH)-, les étapes selon lesquelles on fait réagir un composé de formule (II) 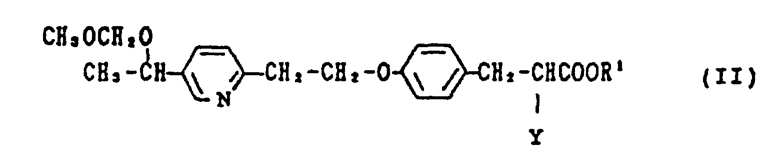 dans laquelle R¹ est un atome d'hydrogène ou un groupe alkyle inférieur et Y est un groupe partant, avec de la thiourée, puis on soumet le composé obtenu de formule (III) 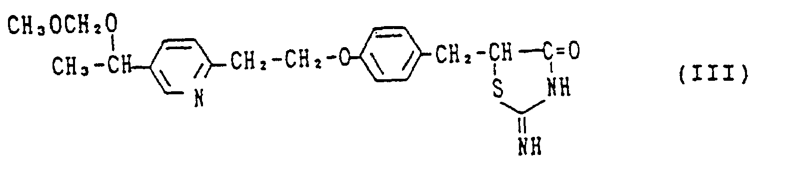 à une hydrolyse acide; ou
(b) pour la production du dérivé de formule générale (I) dans laquelle X est -CH₂- et Q est CH₃CO-, l'oxydation du dérivé de formule générale (I) dans laquelle Q est CH₃CH(OH)-; ou
(c) pour la production d'un dérivé de formule générale (I) dans laquelle X est -CH₂- et Q est CH₃CH(OR)- et R est différent d'un atome d'hydrogène, l'acylation du dérivé de formule générale (I) dans laquelle Q est CH₃CH(OH)-; ou
(d) pour la production du dérivé de formule générale (I) dans laquelle X est ―CH₂- et Q est ―CH₂COOH, l'étape selon laquelle on soumet un composé de formule (V): 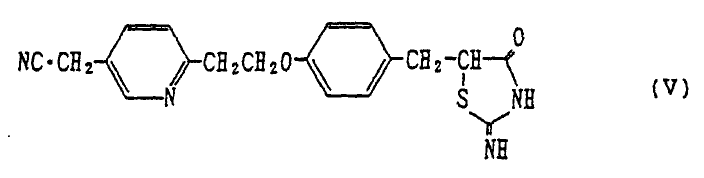 à une hydrolyse acide;
(e) pour la production des dérivés de formule générale (I) dans laquelle X est -CO- et Q est CH₃CH₂-, l'oxydation d'un composé de formule 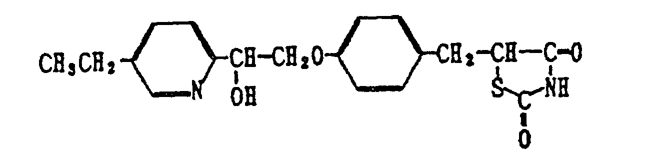 puis, éventuellement, la transformation du dérivé obtenu en un sel.

18. Procédé selon la revendication 17, dans lequel le dérivé de thiazolidinedione est un dérivé selon l'une quelconque des revendications 2 à 16.

19. Composition pharmaceutique pour le traitement du diabète, comprenant un dérivé de thiazolidinedione selon l'une quelconque des revendications 1 à 16 et un support, un excipient ou un diluant pharmacologiquement acceptable.

20. Utilisation d'un dérivé de thiazolidinedione selon l'une quelconque des revendications 1 à 16 pour la fabrication d'un médicament à utiliser dans le traitement du diabète.
